# EUROPEAN PATENT APPLICATION

(11) **EP 0 984 062 A1**
(43) Date of publication of application: **08.03.2000**
(21) Application number: 98116821.4
(22) Date of filing: 04.09.1998
(51) Int. Cl.: C12N 15/12, C12N 15/85, C12N 5/10, C07K 14/505

(54) **Production of human erythropoietin**

(71) Applicant: Cytos Biotechnology AG, 8952 Zürich-Schlieren (CH)
(72) Inventor: Renner, Wolfgang Andreas, Dr., 8006 Zürich (CH)
(74) Representative: Maiwald, Walter, Dr. Dipl.-Chem.

(57) **Abstract**

The present invention relates to the production of recombinant human erythropoietin in mammalian cells, particularly in kidney cells, transformed with an expression vector containing the human erythropoietin gene driven by the myeloproliferative sarcoma virus promoter. Further, this invention relates to the preparation of erythropoietin-producing cells using the tryptophan synthetase gene from *E. coli* as a selectable marker. The invention is also directed to an improved cell culture process using a serum-free and protein-free culture medium.

## Description

The present invention relates to the production of recombinant human erythropoietin in mammalian cells, particularly in kidney cells, transformed with an expression vector containing the human erythropoietin gene driven by the myeloproliferative sarcoma virus promoter. Further, this invention relates to the preparation of erythropoietin-producing cells using the tryptophan synthetase gene from *E. coli* as a selectable marker. The invention is also directed to an improved cell culture process using a serum-free and protein-free culture medium.

Erythropoietin is the principal hormone in the regulation and maintenance of a physiological level of circulating erythropoietin mass, it stimulates cellular differentiation of bone-marrow stem cells at an early stage of erythropoiesis and accelerates the proliferation and maturation of terminally differentiating cells into erythrocytes. Erythropoietin is a glycoprotein with a molecular weight ranging from 34 to 38 kDa of which approximately 40 to 50 % account for carbohydrate moieties. It is produced primarily in the kidneys of adult and the liver of fetal mammals. When the body is in a healthy state wherein tissues receive sufficient oxygenation from the existing number of erythrocytes, erythropoietin is present in the plasma in very low concentrations. This normal low concentration is enough to stimulate replacement of red blood cells which are lost normally through aging. Under conditions of hypoxia when oxygen transport by blood cells in the circulation is reduced, the amount of erythropoietin in the circulation is increased. Hypoxia may be caused by loss of large amounts of blood through hemorrhage, destruction of red blood cells by over-exposure to radiation, reduction in oxygen intake due to high altitudes or prolonged unconsciousness, or various forms of anemia. In the healty organism, a homeostatic mechanism induces the increase of the erythropoietin concentration in the blood depending upon the degree of the anemia to maintain an optimal hematocrit. However, in the case of anemia caused by chronic renal deficiency, erythropoietin can not be produced due to the progressive destruction of kidney mass and kidney function and hence the erythropoietin concentrations in the circulation do not increase.

Erythropoitin is now established as the accepted treatment of various forms of anemia, particularly in chronic renal failure. Further clinical applications of pharmaceutical products containing erythropoietin are the prevention of anemia of patients who receive hemodialysis because of renal insuffiency or after nephrectomy and the promotion of recovery of postoperative patients by stimulating the formation of erythrocytes.

Recombinant human erythropoietin has become available for the treatment of anemia as the first recombinant biomedicine produced in mammalian cells. For example, in Germany, recombinant erythropoietin, produced in a CHO-K1 cell line, is sold by Janssen-Cilag under the trade Erypo®.

The human erythropoietin gene has been isolated and characterized already in 1985 (Lin *et al*. (1985) Proc. Natl. Acad. Sci. USA 82, 7580-7584; Jacobs *et al*. (1985) Nature 313, 806-810). The entire coding region of this gene is contained in a 5.4-kilobase *Hin*dIII-*Bam*HI fragment, the gene containing four introns (1562 base pairs) and five exons (582 base pairs), which encode a 27-amino acid signal peptide for secretion of human erythropoietin and a 166-amino acid mature protein. Further, erythropoietin contains sialic acid, which is required for in vivo activity. Although unglycosylated erythropoietin has in vitro activity, it shows no activity in vivo. This is due to the extremely low half life of the unglycosylated protein in human ciruclation.

Consequently, erythropoietin for therapeutic use has to be manufactured in mammalian cells resulting in similar glycosylation as the native human glycoprotein.

Lin *et al*., supra, describes the production of recombinant human erythropoietin in CHO (Chinese hamster ovary) cells, stably transformed with an expression vector containing the genomic erythropoietin gene driven by the SV40 late promoter, while Jacobs *et al*., supra, discloses the production of erythropoietin in transient expression experiments in COS cells (a cell line derived from monkey cells that contains an integrated segment of SV40 DNA) cells, transfected with a vector containing the human erythropoietin cDNA under control of the adenovirus major late promoter.

Furthermore, European Patent Application No. 0 148 605 describes the isolation and characterization of a monkey erythropoietin cDNA clone and a human genomic clone, and the production of recombinant erythropoietin in COS-1 and CHO cell lines, wherein monkey and human erythropoietin gene expression is under control of the SV40 late promoter.

European Patent Application No. 0 225 231 describes a process for producing erythropoietin in stably transfected mammalian cell lines, wherein an erythropoietin gene is expressed under control of either the adenovirus-2 major late promoter, or the MT-I (metallothionein-I) promoter of mouse.

Further, European Patent Application No. 0 232 034 describes the production of recombinant erythropoietin in human cells, namely in Namalwa cells, using the SV40 promoter to drive expression of erythropoietin and using the neomycin resistance (neo^{r}) gene as the selectable marker.

Similarly, European Patent Application No. 0 236 059 discloses the production of erythropoietin in kidney cells, transfected with an expression vector carrying the human erythropoietin gene under control of the M-MuLV (Moloney murine leukemia virus) LTR promoter, and the neo^{r} gene as the selectable marker.

However, there is still a need in the art for efficient expression systems suitable in large scale production of erythropoietin.

It is thus an object of the present invention to provide an erythropoietin expression vector and a process for the production of recombinant erythropoietin based on said expression vector which provides high level erythropoietin expression in mammalian cells.

Surprisingly, it has been found that using a novel host vector system which is based on the MPSV (myeloproliferative sarcoma virus) promoter and a genomic erythropoietin DNA sequence, the erythropoietin product in mammalian cells represents a very large fraction of up to above 50 % of the total protein in the cell culture supernatant.

According to the present invention, there is, thus, provided a novel expression vector, wherein the human erythropoietin gene is operably linked to the MPSV (myeloproliferative sarcoma virus) promoter. Further, there is provided a process for production of erythropoietin in a mammalian host cell compring the step of culturing under suitable nutrient conditions a mammalian host cell transfected with the novel expression vector.

The myeloproliferative sarcoma virus has been previosly described by Ostertag *et al*. (1980) J. Virol. 33, 573-582; it is known to to transform murine fibroblasts and to cause also changes in cells of the the hemopoietic system. The MPSV promoter, used within this invention, has already been studied by, e.g., Artelt *et al*. (1988) Gene 68, 213-219.

In a preferred embodiment, the process for production of erythropoietin in a mammalian host cell comprises the steps of (1) preparation of an erythropoietin expression vector in which erythropoietin expression is under control of the MPSV promoter, (2) introducing said erythropoietin expression vector into a mammalian host cell, (3) culturing said cell to produce erythropoietin and (4) recovering the erythropoietin from the cell culture medium.

Preferably, the mammalian host cell is a kidney cell, most preferred a baby hamster kidney (BHK) cell, such as BHK 21.

Most of the prior art uses the DHFR selection system, which relies on the fact that the enzyme dihydrofolate reductase (DHFR), coded for by the DHFR gene, can be inhibited by the drug methotrexate, and that cells propagated in media lacking hypoxanthine and thymidine are inhibited or killed by methotrexate. Under appropriate conditions, e.g. minimal concentrations of methotrexate, cells resistant to and able to grow in methotrexate-containing medium, can be obtained. These cells are found to be resistant to methotrexate due to an amplification of the number of their DHFR genes, resulting in increased production of DHFR enzyme. The surviving cells can, in turn, be treated with increasing concentrations of methotrexate, resulting in cell strains containing greater numbers of DHFR genes. The gene of interest, e.g. an erythropoietin gene, carried on the expression vector along with the DHFR gene or transformed with the DHFR gene is frequently found also to be increased in their gene copy number. Thus, the DHFR system is generally used in a combined selection/amplification approach. However, several problems are connected to the use of the DHFR selection amplification system. Due to the high toxity of methotrexate the selection pressure cannot be maintained throughout the manufacturing process. High producers often revert to a mediocre productivity once methotrexate is removed from the culture medium.

Further, the neo marker, another antimetabolite resistance marker frequently used in the prior art, has the principal disadvantage that relatively large amounts of the expensive drug G418 are needed for the selection of transformants in mammalian cells.

Thus, it is a further object of the invention to provide a process for the large scale production of erythropoietin in mammalian cells which overcomes the disadvantages of selectable marker systems previously described in the prior art of erythropoietin production, and which avoids toxic additives.

According to the present invention, there is provided a process for the production of erythropoietin in mammalian host cells, which are selected on the basis of their ability to produce tryptophan.

Most mammalian cells lack the enzymatic pathways required for synthesis of certain, so-called essential amino acids and thus must acquire them from exogenous sources. The absence of even one essential amino acid causes approximately 50 % inhibition of protein sythesis; a basal rate is apparently sustained by turnover of endogenous proteins. In 1988, Hartman and Mulligan (Proc. Natl. Acad. Sci. USA 85, 8047-8051) discovered that tryptophan can be applied as a metabolic marker for selection of transformant mammalian cells. The marker system is based on the tryptophan synthetase gene (trpB) of *E.coli*, which catalyses conversion of indole to tryptophan. However, so far, the trpB gene has never been described as a useful selectable marker in the production of erythropoietin.

Surprisingly, it has been found that by use of trpB as the selectable marker high expression levels of erythropoietin can be observed in selected host cells in the absence of gene amplification.

In a preferred embodiment, the tryptophan synthetase gene is cotransfected with the erythropoietin expression vector to a host cell. However, it is, of course, also possible to transfect the mammalian host cell with a single nucleic acid molecule which contains the DNA sequence coding for human erythropoietin under control of an appropriate promoter, preferably the MPSV promoter, and the trpB gene also under control of an appropriate promoter.

Due to the fact that serum, e.g. fetal bovine serum, which is usually added to the cell culture medium, shows a background of tryptophan, the serum has to be dialyzed to remove tryptophan. Therefore, the trpB selection system is especially useful in large scale production in serum-free medium, wherein tryptophan can be substituted with indole, which is neither toxic nor expensive, more easily.

Therefore, in a prefered embodiment, the process for the production of large amounts of erythropoietin comprises the step of culturing mammalian host cells in a serum-free medium, which lacks exogenous tryptophan. Preferably, the host cell is a kidney cell, preferably a BHK cell, such as BHK 21.

As mentioned above, current manufacturing processes are based on roller bottle cultivation of recombinant mammalian cells, e.g. recombinant Chinese Hamster Ovary cells, which grow in a medium that contains fetal bovine serum. The addition of serum is mandatory to provide the necessary growth factors for cellular proliferation. The presence of this additive, however, is detrimental in several aspects; the major concern relates to product safety. The risk of transferring pathogenic agents from the manufacturing process via the pharmaceutical product to the patient has gained significant consideration, in particular, since the widespread occurrence of bovine spongiform encephalopathy (BSE). Prions, as well as viruses that are not inactivated during downstream processing impose a significant risk at the process. As a consequence of this concern, safety measures necessary for minimising this risk are tremendous, ranging from extensive testing of raw materials (serum) to final product characterization.

In view of the fact that avoidance of any biologically derived material in the manufacturing process not only significantly increases product quality and safety, but at the same time reduces manufacturing costs and simplifies product approval, there is a considerable interest in serum-free and protein-free culture media for the culturing of recombinant mammalian cells.

For example, EP-A-0 148 605 discloses a method for production of erythropoietin from CHO cells in serum-free growth media, being high glucose DMEM supplemented with 0.1 mM non-essential amino acids and L-glutamine, or a 50-50 mixture of high glucose DMEM and Ham's F12 supplemented with 0.05 mM non-essential amino acids and L-glutamine.

Further, European Patent Application No. 0 513 738 describes the cultivation of mammalian cells in a serum-free medium, which contains recombinant insulin from procaryotic cells instead of animal insulin and transferrin, and a water-soluble ferric compound.

EP-A-0 531 911 describes a process for cultivating vero cells in a protein-free medium, wherein culture surfaces from polyvinylformal and polyvinylbutyral are used, and wherein the cells are preferably cultivated as a continous monolayer.

However, in most cases, cultivation in serum-free medium is mentioned only in connection with cell lines which were previously adapted for growth in serum-free media by special adaptive strategies, e.g. the adapted cell line CHO SSF3, described by Zang *et al*. (1995) in Bio/Technology 13, 389-392), or the CHO DUKX cell lineage described by Sinacore *et al*. (1996) in Biotechnology and Bioengineering 52, 518-528.

It has been found that BHK 21 cells grow without adaptation or genetic engineering in the serum- and protein-free medium formulations Turbodoma and Turbodoma HP-1 of the manufacturer Dr. F. Messi Cell Culture Technologies, Buhnrain 14, 8052 Zurich, Switzerland. After transfer of the cells from Eagle's minimum essential medium containing 10 % fetal calf serum and 2 mM L-Glutamin into the Turbodoma medium, cellular proliferation continued without significant decline in viability or growth rate. Moreover this medium formulation allows cultivation of BHK 21 cells alternatively in suspension or attached to carriers. Accordingly, a broad spectrum of reactor systems and process parameters can be applied.

In a preferred embodiment of the invention, the mammalian cell line, which is cultured in the serum-free and protein-free medium of the invention, and which is preferably the BHK 21 cell line, is capable of producing human erythropoietin. Most preferably, the serum-free and protein-free medium contain indole, however no tryptophan, so that only such cells are able to grow which are capable of producing tryptophan due to their tryptophan synthetase activity. This tryptophan synthetase enzymatic activity is accomplished by transfecting the cells with a nucleic acid molecule carrying the trpB gene from *E. coli*. The trpB gene can be under control of any suitable promoter, i.e. a promoter that promotes transcription in a mammalian cell, e.g. viral promoters such as the simian virus 40 (SV40) early promoter, the HIV and the MPSV promoter.

Therefore, in a prefered embodiment, the invention is directed to a process of producing human erythropoietin, which comprises the steps of (1) preparing an erythropoietin expression vector in which erythroppoietin expression is under the control of the MPSV promoter, (2) introducing said erythropoietin expression vector into BHK 21 cells, (3) culturing said cells in a protein-free and serum-free medium, comprising indole, however lacking exogenous tryptophan.

Within this specification, the term 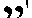 erythropoietin" is meant to define a protein which has the biological activity of erythropoietin, i.e. it is able to stimulate erythropoiesis. The protein may be coded by a genomic DNA sequence or by a cDNA clone. Further, the term 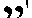 erythropoietin" also comprises a protein fragment, which has the biological activity of naturally-occuring erythropoietin. Preferably, the recombinant protein corresponds fully, or at least substantially, to naturally-occuring human erythropoietin with respect to amino acid sequence and number (166 amino acids in the mature protein), a molecular weight of between approximately 34 to 38 kDa, of which a proportion of about 40% to 50% accounts for carbohydrate moieties.

The gene for human erythropoietin is available , e.g., from the American Type Culture Collection under ATCC No. 40381, as a lambda HE 1 phage lyophilisate.

Further, the term 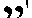 promoter" is meant to define a DNA sequence that precedes a gene (genomic or cDNA clone) in a nucleic acid molecule and provides a site for initiation of the transcription of the gene into mRNA. Accordingly, the term 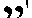 expression vector" defines a nucleic acid molecule, in which a promoter is operably linked to a gene to be expressed, and which enables a host cell to produce the protein encoded by the gene after the vector has been introduced into the host cell. However, in addition to the gene of interest (particularly, an erythropoietin gene), operably fused to a promoter (particularly, the MPSV promoter), the expression vector may contain further promoter sequences or other regulatory sequences such as enhancer sequences, e.g. from SV40, adenovirus, LTR of retrovirus, or of immunoglobulin genes, polyadenylation sequences, or selectable marker sequences. Suitable expression vectors can be obtained using conventional cloning techniques, as e.g. described in the standard manual by Sambrook et al. (1989) Molecular cloning: a laboratory manual, 2^{nd} edition, Cold Spring Harbor Laboratory Press, Cold Spring Harbor, New York.

The term 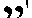 host cell" comprises a cell, which is stably transformed with a heterologous nucleic acid molecule, and hence contains the nucleic acid molecule or at least parts thereof in its genome, and wherein the nucleic acid molecule carries a gene, which is expressed, i.e. transcribed and translated, by the cell, and, optionally, the gene product is secreted by the cell.

To establish recombinant cell lines various transformation techniques may be employed. Preferably, the cells are transfected with the calcium phosphate method (described in Example 2). However, other conventional transfection methods can be used, such as DEAE-dextran mediated transfection techniques, lysozyme fusion, protoplast fusion or erythrocyte fusion, scraping, direct uptake, osmotic or sucrose shock, direct microinjection, indirect microinjection such as via erythrocyte-mediated techniques, or by subjecting the host cells to electric currents.

Generally, by transfection or transformation is meant the transfer of genetic information, and especially the information encoded by a cDNA or genomic erythropoietin clone, into a cell using isolated DNA, RNA, or a synthetic nucleotide polymer by recombinant DNA technology. The transfer of genetic information, particularly of a DNA sequence coding for human erythropoietin, into the cell can be confirmed by cotransfection of the cell with a selectable marker, preferably by using the tryptophan synthetase gene from *E. coli* as the selectable marker.

For the cultivation of the mammalian cells, common culturing methods can be applied, e.g. suspension culture using flasks or dishes usually used for tissue culture, methods of growing the cells in suspension by stirring the culture medium in spinner vessels, methods of growing the cells in hollow fibres into which culture medium is continuously circulated and methods of growing the cells in jar fermentors for cultivation of animal cells.

After the cultivation of the mammalian cells, the recombinant expression product may be recovered in substantially purified form from the culture media using conventional purification techniques, such as affinity chromatography, ion exchange chromatography, hydrophobic interaction chromatography, adhesion chromatography (hydroxyapatite), gel filtration, dye chromatograpy or reversed phase chromatography.

Finally, the biological activity of the recovered erythropoietin can be determined by several different methods. Kurtz and Eckardt reviewed the current assays in Nephron (1989);51 Suppl 1:11-14.

### Description of the Figures

Fig. 1 shows a Western Blot from supernatant of eight selected BHK 21 pMPSVgEPO and eight selected CHO K1:CycE pMPSVgEPO clones.
Fig. 2 shows a silver stained SDS polyacrylamide gel of cell culture supernatant from BHK21 pMPSVgEPO clone 2. Lane 1 shows unconcentrated supernatant, whereas lane 2 shows a ten times concentrated supernatant.
Fig. 3 shows a sample of purified recombinant erythropoietin analysed by Coomassie stained SDS PAGE.

The invention will now be explained in more detail by way of example.

### Example 1

### Preparation of the erythropoietin expression vector pMPSVgEPO

The human erythropoietin gene was obtained from the American Type Culture Collection as a lambda HE 1 phage lyophilisate (Lin *et al*., supra, ATCC Number 40381). Sufficient quantities of lambda phage carrying a fragment containing the genomic DNA for human erythropoietin were prepared by suspending the phage lyophilisate in SM buffer (Sambrook *et al*., supra) and plating the phage on LE 392 *E. coli* cells in LB agar containing 0.3% glucose, 0.075 mM CaCl₂, 0.004 mM FeCl₃, 2 mM MgSO₄ and 1 mg/ml maltose. The phage particles were harvested overnight with 10 ml SM per 15 cm petri dish. The phage DNA of 50 ml of this lysate was purified with the aid of the Qiagen Lambda kit according to manufacturer's instructions (Qiagen, Hilden, Germany). 50 % of the resulting DNA were taken up in 20 µl H₂O and digested with 30 units each of restriction endonucleases *Hin*dIII and *Bam*HI (New England Biolabs (NEB), Schwalbach, Germany). A 5.4 kb fragment was eluted from an 0.8 % agarose gel with the aid of the Gene Clean Kit of Bio 101 (Vista CA, USA). This 5.4 kb fragment contains the entire coding of the human erythropoietin gene, which contains four intervening sequences and five exons (cf. also Lin *et al*., supra). The vector pMPSVHE (Artelt *et al*, supra), carrying the MPSV promoter, was digested with restriction endonuclease *Hin*dIII and *Bam*HI according to manufacturer's instructions (NEB), and the linearized vector DNA was gel purified using the Gene Clean Kit Bio 101. Approximately 0.5 µg of the vector DNA and 2µg of the purified erythropoietin gene insert were ligated using T4 DNA Ligase (NEB) and the product was transformed into RbCl₂ competent DH5alpha *E. coli* cells (Sambrook *et al*., supra). After identification of a colony with correct insert by restriction analysis and large scale preparation of plasmid DNA by use of the Qiagen Midiprep Kit, an 575 bp fragment was deleted from the 5' end of the erythropoietin gene insert by digesting the vector with the restriction enzymes *Hin*dIII and *Bst* EII (NEB) in the presence of buffer 2 (NEB); see also Lin *et al*., supra, for a restriction map of the erythropoietin gene insert. The sticky ends were filled with T7 DNA polymerase (NEB) at 37 °C for 30 min. in the presence of all 4 nucleotides. The product of this fill-in reaction was ligated at 16 °C for 30 min. in the presence of T4 DNA Ligase (NEB) and subsequently transformed into RbCl₂ competent DH5alpha *E. coli* cells. After restriction analysis, an *E. coli* clone with the correct vector construct was identified and larger quantities of said vector DNA were prepared with the aid of the Qiagen Midiprep Kit according to manufacturer's instruction. The resulting vector, carrying the genomic erythropoietin gene under control of the MPSV promoter, was designated pMPSVgEPO.

### Example 2

### Stable transfection of BHK 21 and CHO K1:cycE cells with the pMPSVgEPO vector

The tryptophan synthetase gene (trpB) of *E. coli* was used for stable selection of the pMPSVgEPO vector. In this system, transfected cells are selected by their ability to convert indole into the essential amino acid tryptophan by enzymatic activity of the gene product of the tryptophan synthetase gene (trpB).

Prior to CaPO₄ transfection, the expression vector was linearized with the restriction enzyme *Aat*II, and the trpB selection plasmid pSV2trpB (described in Hartman and Mulligan, supra) was linearized with the restriction endonuclease *Eco*RI. A mixture of 5.7 µg of pMPSVgEPO and 0.3 µg pSV2trpB was prepared in 30 µl H₂O. 30 µl of 1 M CaCl₂ solution were added and mixed. 60µl of phosphate solution (50 mM HEPES, 280 mM NaCl, 1.5 mM Na₂HPO4, pH 7.05, sterile filtered, not autoclaved) were added and vortexed for 5 seconds. After incubation for 25 seconds at room temperature, the precipitate was transferred to a polypropylene tube with 2 ml of Turbodoma medium with 2 % fetal calf serum (FCS).

The medium of 80% confluent BHK 21 and CHO K1:cycE cell cultures (Renner et al. Biotech. Bioeng. 47, 476-482, 1995) in six well plate were removed and the cells were washed once with serum-free Turbodoma medium. After addition of the 2 ml medium with the precipitate, the cell culture was incubated for 5 hours at 37 °C and 5 % CO₂. The precipitate was removed and Turbodoma medium containing 15 % glycerol (Sigma) and 2 % FCS were added. After 30 seconds the medium with the glycerol was removed and 5 ml of medium containing 10 % FCS were added, and the plate was rocked a few times. The medium was replaced with 3 ml of fresh serum-free medium and after 18 hours the cells were transferred into a 15 cm cell culture petri dish filled with 50 ml selection medium containing 300 µM indole instead of tryptophan plus 5 % of dialysed fetal calf serum (Life Technologies). After incubation for ten days, primary clones were visible and picked after overlaying the culture with 0.8 % agarose. A 1.6 % agarose solution was prepared in PBS without Mg and Ca, autoclaved and cooled to 42 °C. This solution was mixed in a 1:1 ratio with prewarmed medium (42 °C) and the plate was overlayed with 30 ml of this solution. After gelling at room temperature, the colonies were picked with a pipette. Residual cells that sticked to the cell culture plastic were detached with trypsin.

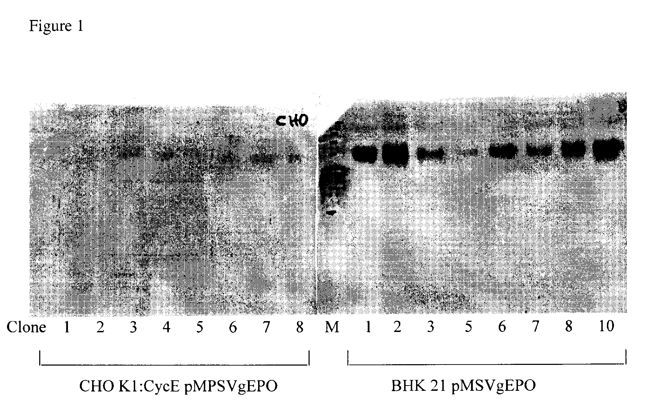

Primary clones were grown in a 24 well plate and then in T25 flasks in serum-free Turbodoma medium. The productivity of eight selected BHK 21 and CHO K1:cycE cell clones were assayed in Western Blot analysis. The productivity in all BHK 21 cell clones was at least ten fold higher than that of CHO K1:cycE cells as compared in Western Blot analysis (see figure 1). All BHK 21 clones produced recombinant erythropoietin in significant amounts.

The two best producers (clone 2 and 10) were tested in different cell culture systems in suspension and on microcarriers. Examples 3 and 4 give details.

### Example 3

### Production of recombinant human erythropoietin in suspension culture

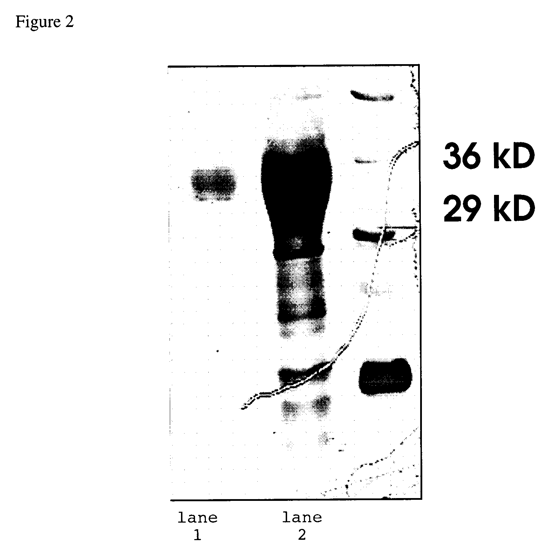

BHK21 pMPSVgEPO, clone 2 was grown in Spinner flasks (Integra Bioscience) in suspension in serum-free and protein-free Turbodoma medium. 20 million cells grown in T-flasks were detached with Cell Dissociation Solution (Sigma) and inoculated in 200 ml of Turbodoma medium supplemented with 0.5 g/l Pluronic F68 (Sigma) in a spinner flask. Culture conditions were: 37°C, 5% CO2, rotation of 20 rpm. Starting with a density of 100,000 cells/ml, the cells grew within 4 days to a cell density of approximately 1 million cells/ml. After seven days the cell culture was harvested and the culture liquid was centrifuged for three minutes at 250 g. The supernatant was analysed by SDS PAGE and a strong band was detected at a molecular weight of approximately 34 - 38 kD (figure 2). This band was confirmed in Western blot analysis to be crossreactive with an anti erythropoietin antibody (Research Diagnostics, USA). As can be seen in figure 2, the product represents about 50 % of the total protein in the supernatant.

### Example 4

### Production of recombinant human erythropoietin in microcarrier culture

BHK21 pMPSVgEPO, clone 2 was grown attached on microcarriers in Spinner flasks (Integra Bioscience) in serum-free and protein-free Turbodoma HP-1 medium (F. Messi Cell Culture Technologies). 20 million cells grown in T-flasks were detached with Cell Dissociation Solution (Sigma) and inoculated with 0.6 g of Cytodex 1 microcarriers (Pharmacia) in 25ml of Turbodoma HP-1 medium during 5 hours in the presence of 5% CO2 and at 37°C. The microcarriers were prepared as described in the manufacturer's instructions. The culture was transferred into a spinner flask with 200 ml Turbodoma HP-1 medium supplemented with 0.5 g/l Pluronic F68 (Sigma). Culture conditions were: 37°C, 5% CO2, rotation of 20 rpm. Starting with a density of 100,000 cells/ml, the cells grew within 4 days to confluency. At day 4 the microcarriers were allowed to settle and 70% of the medium were replaced with fresh medium. At days 5 and 6 each, another 40 % of the medium were replaced with fresh medium and at day 7 the culture was harvested. The supernatant contained recombinant human erythropoietin at a concentration of 10 mg/l as determined by ELISA (EPO ELISA, Boehringer Mannheim). This recombinant erythropoietin product was purified as described in example 5.

### Example 5

### Purification of recombinant human erythropoietin

This example relates to a purification of human recombinant EPO from cell culture supernatant fluid. This process comprising the following steps:
A. Dye Chromatography on Blue Sepharose
B. Hydrophobic Interaction Chromatography on Butyl-Toyopearl
C. Chromatography on Hydroxylapatite
D. Anion Exchange Chromatography on Resource Q
revealed in a polypeptide component at about 34,000 MW when analyzed by SDS-PAGE according Laemmli, U.K. and stained with Coomassie or Silver.

### A. Dye Chromatography

The pH of the cell-free culture supernatant was adjusted to pH 5.0 with acetic acid and was filtered through a 0.45um filter at 4°C to 10°C. The filtrate, in this example 200ml, was applied onto a 5ml blue sepharose CL-6B (Pharmacia) column previously equilibrated with 20mM sodium acetate, 5mM CaCl₂, and 100mM NaCl at pH 5.0 with 4ml/min at 10°C. The gel was washed with 5CV of 20mM sodium acetate, 5mM CaCl₂ and 250mM NaCl at pH 5.0 and 5CV of 20mM Tris-HCl, 5mM CaCl₂ at pH 6.5, respectively. The protein was eluted with 2CV of 100mM Tris-HCl, 5mM CaCl₂ and 1M NaCl at pH 9.0.

### B. Hydrophobic Interaction Chromatography

The eluate from the blue sepharose column was brought to pH 6.9 with 1N HCl immediatedly after chromatography, and to 10% isopropanol before loading. A 5ml column with Butyl-Toyopearl (TosoHaas) was run at room temperature and equilibrated with 20mM Tris-HCl, 5mM CaCl₂, 750mM NaCl, 10% isopropanol at pH 6.9. After loading the protein sample the column was washed with 20mM Tris-HCl, 5mM CaCl₂, 750mM NaCl and 19% isopropanol at pH 6.9. The proteins, predominantly EPO, were eluted with 20mM Tris-HCl, 5mM CaCl₂, 750mM NaCl and 27% isopropanol at pH 6.9. The fraction was diluted one third with 20mM Tris-HCl, 5mM CaCl₂ at pH 6.9.

### C. Chromatography on Hydroxylapatite

A 5ml column with Hydroxylapatite Ultrogel was run at room temperature and equilibrated with 20mM Tris-HCl, 5mM CaCl₂, 250mM NaCl, 9% isopropanol, pH 6.9. After loading the diluted eluate from the previous step the column was washed with 5CV of 10mM Tris-HCl, 5mM CaCl₂, pH 6.8. The protein was eluted with 20mM Tris-HCl, 5mM CaCl₂, 750mM NaCl and 27% isopropanol, pH 6.9. The fraction was diluted one third with 10mM Tris-HCl, 10mM sodium phosphate and 5mM CaCl₂, pH 6.9.

### D. Anion Exchange Chromatography

For a final purification step a 1ml Resource Q column (Pharmacia) was applied at 15°C. The column was equilibrated with 10mM sodium phosphate, pH 7.0. The eluate from the hydroxyapatite column was loaded, followed by a washing step with equilibration buffer for 2CV. Finally, the bound erythropoietin was released by elution with 10mM sodium phosphate, 250mM NaCl at pH 7.0 where traces of DNA remain on the column. The protein fraction was concentrated 10 times by means of an ultrafree-15 5k ultrafilter (Millipore) and diluted with 2 volumes of 10mM sodium phosphate, pH 7.0.

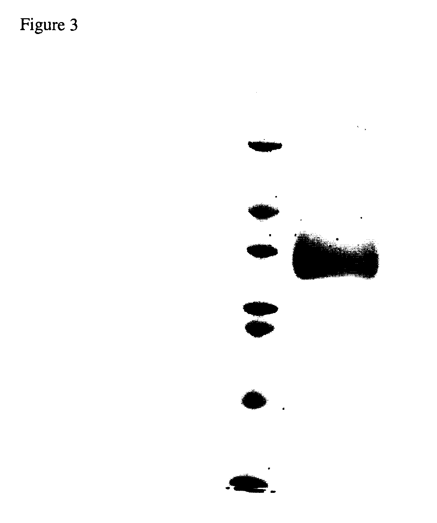

A sample of the resulting erythropoietin product has been analysed on Coomanssie stained SDS PAGE. 70 µg of purified recombinant human erythropoietin were loaded, and, as shown in figure 3 the product is essentially free of any host cell derived proteins.

While the present invention has been described in conjunction with preferred embodiments, one of ordinary skill after reading the foregoing specification will be able to effect various changes, substitutions or equivalents, and other alterations to the methods set forth herein.

## Claims

1. A eucaryotic expression vector comprising a DNA sequence that codes for human erythropoietin under control of the myeloproliferative sarcoma virus promoter.

2. The expression vector according to claim 1, wherein the DNA sequence originates from a genomic clone.

3. A mammalian host cell transformed or transfected with an expression vector according to claim 1 or 2.

4. Transformed or transfected kidney cell according to claim 3.

5. Transformed or transfected baby hamster kidney cell, preferably BHK 21 cell, according to claim 3 or 4.

6. A process for production of human erythropoietin in a mammalian cell, comprising the step of culturing under suitable nutrient conditions a mammalian host cell according to one of claims 3 to 5.

7. The process according to claim 6, further comprising the steps of preparation of an erythropoietin expression vector wherein human erythropoietin expression is under control of the MPSV promoter, introducing said erythropoietin expression vector into a host cell, and recovering the erythropoietin from the cell culture medium after cultivation of the transformed or transfected host cell.

8. A process for production of human erythropoietin in a mammalian host cell, comprising the step of culturing under suitable nutrient conditions a mammalian host cell transfected or transformed with an erythropoietin expression vector and selected by its ability to produce tryptophan.

9. Process according to claim 8 wherein the host cell contains the erythropoietin expression vector of claim 1 or 2.

10. Process according to claim 8 or 9 wherein the host cell is a kidney cell, preferably a BHK 21, cell.

11. Process according to claim 6 further comprising the step of selecting the erythropoietin-producing mammalian host cell by its ability to produce tryptophan.

12. Process according to claim 8 or 11, wherein the host cell is able to produce tryptophan due to expression of the tryptophan synthetase (trpB) gene of *Escherichia coli*.

13. The process according to one of claims 6 to 12 comprising the step of culturing the host cell in a serum-free and protein-free culture medium.

14. A process of culturing a mammalian host cells, preferably a kidney cell and most preferably a BHK 21 cell, in the Turbodoma medium.

15. Process according to claim 14, wherein the cultured host cell produces erythropoietin.

16. The process according to claim 14 or 15, wherein the host cell is selected by its ability to produce tryptophan.
